# EUROPEAN PATENT APPLICATION

(11) **EP 3 118 193 A1**
(43) Date of publication of application: **18.01.2017**
(21) Application number: 14885180.1
(22) Date of filing: 26.09.2014
(51) Int. Cl.: C07D 213/24, C09K 11/06, G01N 21/78, G01N 33/58

(54) **TWO-PHOTON-ABSORBING COMPOUND**

(30) Priority: 11.03.2014 JP 2014047329
(71) Applicant: OTSUKA ELECTRONICS CO., LTD., Hirakata-shi Osaka 573-1132 (JP)
(72) Inventor: SUZUKI, Yasutaka, Yamaguchi-shi Yamaguchi 753-8512 (JP); KAWAMATA, Jun, Yamaguchi-shi Yamaguchi 753-8512 (JP); MORITOMO, Hiroki, Yamaguchi-shi Yamaguchi 753-8512 (JP); TOMINAGA, Makoto, Yamaguchi-shi Yamaguchi 753-8512 (JP); KONISHI, Gen-ichi, Tokyo 152-8550 (JP); NIKO, Yosuke, Kochi-shi Kochi 780-8520 (JP)
(74) Representative: Hamer, Christopher K.
(86) International application number: PCT/JP2014/004948
(87) International publication number: WO 2015/136583

(57) **Abstract**

An object of the present invention is to provide a two-photon-absorbing compound which is excellent in water-solubility, is excited by two-photon absorption in a near-infrared wavelength region, and emits a red fluorescence.

A compound represented by formula (1) [wherein X¹ and X² are the same or different, and each represents the following formula (2) (wherein R¹ represents a C1-C3 alkyl group, Z⁻ represents a counter anion to a pyridinium cation, and a wavy line represents a covalent bond to Y), and Y represents a condensed polycyclic group having 2 to 4 rings] is excellent in water-solubility, is excited by two-photon absorption in a near-infrared wavelength region, and emits a red fluorescence.

X¹-Y-X² (1)

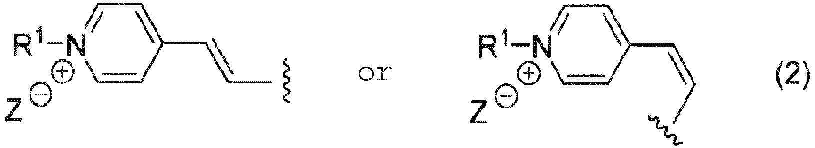

## Description

### Technical Field

The present invention relates to a novel two-photon-absorbing compound, and more specifically to a compound which is excellent in water-solubility, absorbs two photons in a near-infrared wavelength region, and emits a red fluorescence. In addition, the present invention also relates to a fluorescent probe composition comprising the two-photon-absorbing compound, and a fluorescent probe composition for use in bioimaging.

### Background Art

Two-photon absorption means that, considering light as a photon, the state of molecules is excited by simultaneously absorbing two photons, so that it makes a transition to a higher energy level. Such two-photon absorption is a nonlinear phenomenon in which the probability of generation of the two-photon absorption is proportional to the square of the intensity of light. Accordingly, since light absorption is observed only when the light intensity is high, if light is concentrated with lens, absorption is allowed to take place only around a focal point at which light intensity is high. Moreover, since even light with low energy can be excited to a high transition energy, an excited state can be created, for example, by concentrating near-infrared light with lens to create a two-photon phenomenon and then subjecting to two-photon irradiation, molecules that are not excited by such near-infrared light.

The characteristics in which two-photon absorption takes place only around a focal point are applied, for example, to bioimaging (Non-Patent Document 1). Bioimaging means a technique of grasping the distribution/localization of a protein or the like at a level of cells/tissues or an individual body, and then analyzing the movement thereof in the form of an image, and this is a useful means for pathological elucidation, diagnosis and the like of a subject that is in a pathological condition. If two-photon absorption is utilized in such bioimaging, a three-dimensional image can be obtained by scanning the position of a focal point with respect to a measurement sample.

In order to obtain an image of the deep part of a sample by bioimaging, the light in a long wavelength region around a near-infrared region is considered to be preferable, since light absorption and scattering in the sample are large in a visible light region and it causes poor permeability. Thus, two-photon absorption excited by the light in the long wavelength region is suitable for the imaging of the deep part of a sample.

In bioimaging involving two-photon absorption, there is applied, for example, a method which comprises adding a fluorescent substance used as a two-photon absorption material to a sample as a measurement subject, then allowing a target biomolecule to interact with the fluorescent substance, and then applying thereto a light obtained by concentrating a near-infrared light with lens, to detect light emission from the fluorescent substance, so as to obtain an image. This method has been known as "two-photon fluorescence bioimaging."

In order to obtain an image of the deep part of a sample by two-photon fluorescence bioimaging, light emission around a near-infrared region is desirable, so that two-photon absorption takes places by a near-infrared light and the generated fluorescence can permeate into the sample. Hence, it has been desired to discover a compound in which two-photon absorption efficiently takes place by the light around a near-infrared region and light emission takes place around the near-infrared region.

For the efficient occurrence of two-photon absorption, it is necessary to select a compound whose two-photon absorption cross-section (GM) showing such high efficiency is large. For that purpose, a compound whose π-electron conjugated system is extended or the like is considered to be appropriate. However, as the π-electron conjugated system of a compound is extended, the solubility of the compound in a polar solvent such as water or alcohol becomes deteriorated, and thus, upon performing bioimaging, it becomes difficult to add such a compound to an organism sample as a measurement subject and to distribute it into the living body. Accordingly, it has been desired to discover a two-photon-absorbing compound having the property of efficiently causing two-photon absorption, the property of emitting a red light, and the property of exhibiting water-solubility.

### Prior Art Documents

### Non-Patent Documents

Non-Patent Document 1: Biochemistry 46 (2007) 9674

### Summary of the Invention

### Object to be Solved by the Invention

An object of the present invention is to provide a two-photon-absorbing compound which is excellent in water-solubility, is excited by two-photon absorption in a near-infrared wavelength region, and emits a red fluorescence.

### Means to Solve the Object

As a result of intensive studies directed towards achieving the aforementioned object, the present inventors have found that a condensed polycyclic group compound having 2 to 4 rings, having two N-alkylpyridinylethenyl groups as substituents, is a two-photon-absorbing compound which is excellent in water-solubility, is excited by two-photon absorption in a near-infrared wavelength region, and emits a red fluorescence.

Specifically, the present invention relates to:
(1) A compound represented by the following formula (1):

   X¹-Y-X² (1)

   [wherein X¹ and X² are the same or different, and each represents the following formula (2): 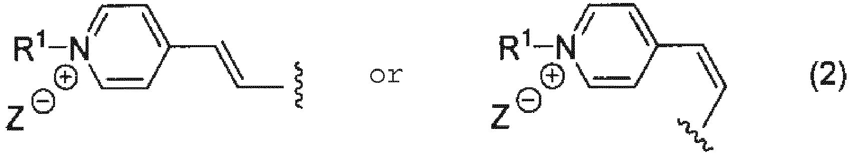 (wherein R¹ represents a C1-C3 alkyl group, Z⁻ represents a counter anion to a pyridinium cation, and a wavy line represents a covalent bond to Y), and Y represents a condensed polycyclic group having 2 to 4 rings];
(2) The compound according to the above (1), wherein Y is any one of condensed polycyclic groups represented by the following formulae: 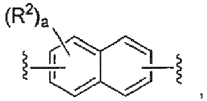 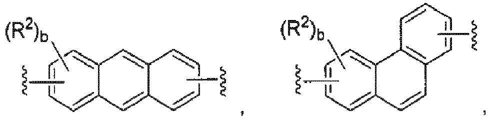 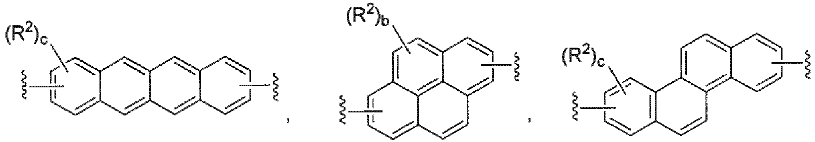 (wherein R² represents an electron-donating group, a represents an integer of 0 to 6, b represents an integer of 0 to 8, and c represents an integer of 0 to 10; when a, b, or c is an integer of 2 or more, R² is identical to or different from one another; and a wavy line represents a covalent bond to X¹ and X²) ;
(3) The compound according to the above (2), wherein Y is any one of condensed polycyclic groups represented by the following formulae: 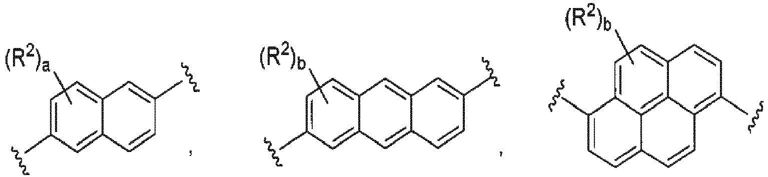 (wherein R² represents an electron-donating group, a represents an integer of 0 to 6, and b represents an integer of 0 to 8; when a or b is an integer of 2 or more, R² is identical to or different from one another; and a wavy line represents a covalent bond to X¹ and X²) ;
(4) The compound according to the above (2) or (3), wherein the electron-donating group is one or more selected from the group consisting of a hydroxyl group, a C1-C10 alkyl group, a C1-C10 alkoxy group, an amino group, an alkyl group having an ether bond, and an alkoxy group having an ether bond;
(5) The compound according to any one of the above (1) to (4), wherein the counter anion is a halide ion or sulfonate;
(6) The compound according to any one of the above (1) to (5), wherein the compound has two-photon absorption in a wavelength region of 600 to 1200 nm;
(7) The compound according to any one of the above (1) to (6), wherein the compound emits fluorescence in a wavelength region of 600 to 900 nm;
(8) A fluorescent probe composition comprising one or more of the compounds according to any one of the above (1) to (7) ;
(9) A fluorescent probe composition for use in bioimaging, comprising one or more of the compounds according to any one of the above (1) to (7); and
(10) A fluorescent biomolecule comprising a biomolecule to which the compound according to any one of the above (1) to (7) chemically binds.

### Effect of the Invention

Since the compound of the present invention is excited by two-photon absorption in a near-infrared wavelength region, emits a red fluorescence, and also has water-solubility, it can be used as a fluorescent probe, and the bioimaging of an organism, such as cells, tissues, an organ and an individual body, can be carried out. Moreover, since the present compound emits a red fluorescence that easily passes through an organism, it becomes possible to achieve the imaging of the deep part of an organism.

### Brief Description of Drawings

[Figure 1] Figure 1 shows the ¹HNMR chart of naphthalene derivative (I).
[Figure 2] Figure 2 shows the ¹HNMR chart of anthracene derivative (II).
[Figure 3] Figure 3 shows the ¹HNMR chart of pyrene derivative (III).
[Figure 4] Figure 4 shows the ultraviolet-visible absorption spectrum of naphthalene derivative (I).
[Figure 5] Figure 5 shows the ultraviolet-visible absorption spectrum of anthracene derivative (II).
[Figure 6] Figure 6 shows the ultraviolet-visible absorption spectrum of pyrene derivative (III).
[Figure 7] Figure 7 shows the ultraviolet-visible absorption spectrum of benzene derivative (IV).
[Figure 8] Figure 8 shows the fluorescence spectrum of naphthalene derivative (I).
[Figure 9] Figure 9 shows the fluorescence spectrum of anthracene derivative (II).
[Figure 10] Figure 10 shows the fluorescence spectrum of pyrene derivative (III).
[Figure 11] Figure 11 shows the fluorescence spectrum of benzene derivative (IV).
[Figure 12] Figure 12 shows the two-photon absorption cross-section spectrum of naphthalene derivative (I).
[Figure 13] Figure 13 shows the two-photon absorption cross-section spectrum of anthracene derivative (II).
[Figure 14] Figure 14 shows the two-photon absorption cross-section spectrum of pyrene derivative (III).
[Figure 15] Figure 15 shows the two-photon absorption cross-section spectrum of benzene derivative (IV).
[Figure 16] Figure 16 shows a schematic view of the optical system of two-photon excitation fluorescence microscopy.
[Figure 17] Figure 17 shows a two-photon excitation fluorescence microscopic image of Hek293 cells stained with naphthalene derivative (I).
[Figure 18] Figure 18 shows a two-photon excitation fluorescence microscopic image of Hek293 cells stained with anthracene derivative (II).
[Figure 19] Figure 19 shows a two-photon excitation fluorescence microscopic image of Hek293 cells stained with pyrene derivative (III).

### Mode of Carrying Out the Invention

### (Compound)

The compound of the present invention is a compound represented by the following formula (1).

X¹-Y-X² (1)

[wherein X¹ and X² are the same or different, and each represents the following formula (2): 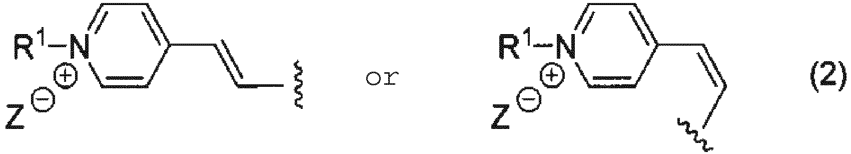 (wherein R¹ represents a C1-C3 alkyl group, Z⁻ represents a counter anion to a pyridinium cation, and a wavy line represents a covalent bond to Y), and Y represents a condensed polycyclic group having 2 to 4 rings].

The above described C1-C3 alkyl group means a linear or branched alkyl group containing 1 to 3 carbon atoms, and examples of the C1-C3 alkyl group include a methyl group, an ethyl group, an n-propyl group, and an isopropyl group.

Examples of the above described counter anion to a pyridinium cation include: a halide ion such as a chlorine ion, a bromine ion, or an iodine ion; a sulfonate such as a methanesulfonate, p-toluenesulfonate, trifluoromethanesulfonate, or trifluoroethanesulfonate; and hexafluoroantimonate, hexafluorophosphate, and tetrafluoroborate. Among others, a halide ion and a sulfonate are preferable.

The above described condensed polycyclic group having 2 to 4 rings indicates any one of condensed polycyclic groups represented by the following formulae: 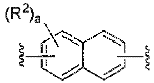 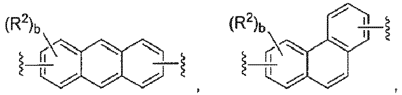 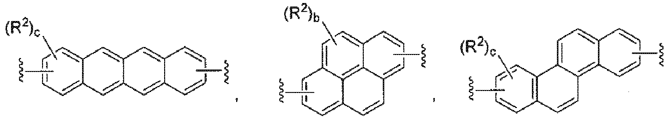 (wherein R² represents an electron-donating group, a represents an integer of 0 to 6, b represents an integer of 0 to 8, and c represents an integer of 0 to 10; when a, b, or c is an integer of 2 or more, R² is identical to or different from one another; and a wavy line represents a covalent bond to X¹ and X²).

Among the above described condensed polycyclic groups represented by Y, condensed polycyclic groups represented by the following formulae are preferable: 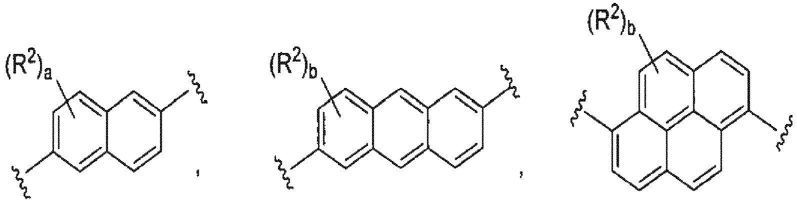 (wherein R², a, b, and a wavy line are the same as those described above).

The above described electron-donating group means a group having the effect of increasing the electron density of a condensed polycyclic group, and examples of the electron-donating group include a hydroxyl group, a C1-C10 alkyl group, a C1-C10 alkoxy group, an amino group, an alkyl group having an ether bond, and an alkoxy group having an ether bond.

The above described C1-C10 alkyl group means a linear or branched alkyl group containing 1 to 10 carbon atoms, and examples of the C1-C10 alkyl group include a methyl group, an ethyl group, a propyl group, an isopropyl group, an n-butyl group, an isobutyl group, a tert-butyl group, an n-pentyl group, an n-hexyl group, an n-heptyl group, an n-octyl group, a nonyl group, an isononyl group, and a decyl group.

Examples of the above described C1-C10 alkoxy group include a methoxy group, an ethoxy group, an n-propoxy group, an isopropoxy group, an n-butoxy group, an isobutoxy group, a sec-butoxy group, a tert-butoxy group, an n-pentyloxy group, an n-hexyloxy group, an n-heptyloxy group, an isoheptyloxy group, a tert-heptyloxy group, an n-octyloxy group, an isooctyloxy group, a tert-octyloxy group, and a 2-ethylhexyloxy group.

The above described amino group means a functional group represented by -NH₂, -NHR³, or -NR³R³'. Herein, R³ and R³' each represents a C1-C10 alkyl group, such as a methyl group, an ethyl group, a propyl group, an isopropyl group, an n-butyl group, an isobutyl group, a tert-butyl group, an n-pentyl group, an n-hexyl group, an n-heptyl group, an n-octyl group, a nonyl group, an isononyl group, or a decyl group; a C3-C6 cycloalkyl group, such as a cyclopropyl group, a cyclobutyl group, a cyclopentyl group, or a cyclohexyl group; a phenyl group; or the like.

The alkyl group having an ether bond and the alkoxy group having an ether bond mean an alkyl group and an alkoxy group, each of which has one or more ether bonds, and examples of the alkyl group and the alkoxy group include -CH₂OCH₃, -OCH₂OCH₃, -CH₂OCH₂CH₃, -OCH₂OCH₂CH₃,-(CH₂)₂OCH₂CH₃, -O(CH₂)₂OCH₂CH₃, - (CH₂)₂O(CH₂)₂CH₃,-O(CH₂)₂O(CH₂)₂CH₃, - (CH₂)₃O(CH₂)₂CH₃, -O(CH₂)₃O(CH₂)₂CH₃,-(CH₂)₂O(CH₂)₂O(CH₂)₂CH₃, and -O(CH₂)₂O(CH₂)₂O(CH₂)₂CH₃.

Preferred examples of the above described electron-donating group include a methyl group, an ethyl group, a propyl group, an isopropyl group, an n-butyl group, an isobutyl group, a tert-butyl group, a methoxy group, an ethoxy group, an n-propoxy group, an isopropoxy group, an n-butoxy group, an isobutoxy group, a sec-butoxy group, a tert-butoxy group, a hydroxyl group, -NH₂, a methylamino group, a dimethylamino group, an ethylamino group, a diethylamino group, -CH₂OCH₃, -OCH₂OCH₃, -(CH₂)₂O(CH₂)₂CH₃, and -O(CH₂)₂O(CH₂)₂CH₃.

The compound represented by formula (1) is preferably a compound that is excited by two-photon absorption in a region ranging from a visible region to an infrared region, and preferably a compound in which the two-photon absorption takes place in a wavelength region of 600 to 1200 nm. In addition, among the compounds represented by formula (1), compounds emitting fluorescence in a wavelength region of 600 to 900 nm by two-photon absorption are preferable. Moreover, among the compounds represented by formula (1), compounds having a two-photon absorption cross-section of 200 GM or more (wherein 1 GM = 10⁻⁵⁰ cm⁴ s molecule⁻¹ photon⁻¹) are preferable, and compounds having a two-photon absorption cross-section of 500 GM or more are more preferable.

Specific examples of the compound represented by formula (1) include the compounds shown in Table 1 to Table 3. The number on the aromatic ring indicates the carbon number of the aromatic ring.

**[Table 1]**

| Table 1 | | | | | | |
|---|---|---|---|---|---|---|
| 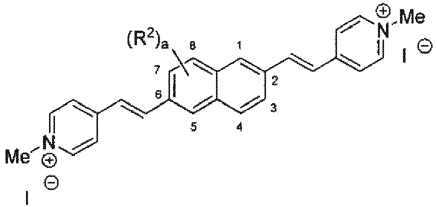 | | | | | | |

| Compound No. | Carbon number | | | | | |
|---|---|---|---|---|---|---|
| | 1 | 3 | 4 | 5 | 7 | 8 |
| 1 | H | H | H | H | H | H |
| 2 | H | Me | H | H | Me | H |
| 3 | H | Et | H | H | Et | H |
| 4 | H | Pr | H | H | Pr | H |
| 5 | H | i-Pr | H | H | i-Pr | H |
| 6 | H | n-Bu | H | H | n-Bu | H |
| 7 | H | i-Bu | H | H | i-Bu | H |
| 8 | H | t-Bu | H | H | t-Bu | H |
| 9 | H | MeO | H | H | MeO | H |
| 10 | H | EtO | H | H | EtO | H |
| 11 | H | PrO | H | H | PrO | H |
| 12 | H | i-PrO | H | H | i-PrO | H |
| 13 | H | n-BuO | H | H | n-BuO | H |
| 14 | H | i-BuO | H | H | i-BuO | H |
| 15 | H | s-BuO | H | H | s-BuO | H |
| 16 | H | t-BuO | H | H | t-BuO | H |
| 17 | H | OH | H | H | OH | H |
| 18 | H | -NH₂ | H | H | -NH₂ | H |
| 19 | H | -NHMe | H | H | -NHMc | H |
| 20 | H | -NMc₂ | H | H | -NMe₂ | H |
| 21 | H | -NHEt | H | H | -NHEt | H |
| 22 | II | -NEt₂ | H | H | -NEt₂ | H |
| 23 | H | -CH₂OCH₃ | H | H | -CH₂OCH₃ | H |
| 24 | H | -OCH₂OCH₃ | H | H | -OCH₂OCH₃ | H |
| 25 | H | -(CH₂)₂O(CH₂)₂CH₃ | H | H | -(CH₂)₂O(CH₂)₂CH₃ | H |
| 26 | H | -O(CH₂)₂O(CH₂)₂CH₃ | H | H | -O(CH₂)₂O(CH₂)₂CH₃ | H |
| 27 | H | Me | H | H | H | H |
| 28 | H | Et | H | H | H | H |
| 29 | H | Pr | H | H | H | H |
| 30 | H | i-Pr | H | H | H | H |
| 31 | H | n-Bu | H | H | H | H |
| 32 | H | i-Bu | H | H | H | H |
| 33 | H | t-Bu | H | H | H | H |
| 34 | H | MeO | H | H | H | H |
| 35 | H | EtO | H | H | H | H |
| 36 | H | PrO | H | H | H | H |
| 37 | H | i-PrO | H | H | H | H |
| 38 | H | n-BuO | H | H | H | H |
| 39 | H | i-BuO | H | H | H | H |
| 40 | H | s-BuO | H | H | H | H |
| 41 | H | t-BuO | H | H | H | H |
| 42 | H | OH | H | H | H | H |
| 43 | H | -NH₂ | H | H | H | H |
| 44 | H | -NHMe | H | H | H | H |
| 45 | H | -NMe₂ | H | H | H | H |
| 46 | H | -NHEt | H | H | H | H |
| 47 | H | -NEt₂ | H | H | H | H |
| 48 | H | -CH₂OCH₃ | H | H | H | H |
| 49 | H | -OCH₂OCH₃ | H | H | H | H |
| 50 | H | -(CH₂)₂O(CH₂)₂CH₃ | H | H | H | H |
| 51 | H | -O(CH₂)₂O(CH₂)₂CH₃ | H | H | H | H |

**[Table 2]**

| Table 2 | | | | | | | | |
|---|---|---|---|---|---|---|---|---|
| 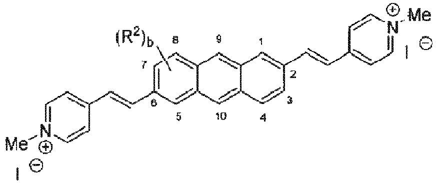 | | | | | | | | |

| Compound No. | Carbon number | | | | | | | |
|---|---|---|---|---|---|---|---|---|
| | 1 | 3 | 4 | 5 | 7 | 8 | 9 | 10 |
| 52 | H | H | H | H | H | H | H | H |
| 53 | H | Me | H | H | Me | H | H | H |
| 54 | H | Et | H | H | Et | H | H | H |
| 55 | H | Pr | H | H | Pr | H | H | H |
| 56 | H | i-Pr | H | H | i-Pr | H | H | H |
| 57 | H | n-Bu | H | H | n-Bu | H | H | H |
| 58 | H | i-Bu | H | H | i-Bu | H | H | H |
| 59 | H | t-Bu | H | H | t-Bu | H | H | H |
| 60 | H | MeO | H | H | MeO | H | H | H |
| 61 | H | EtO | H | H | EtO | H | H | H |
| 62 | H | PrO | H | H | PrO | H | H | H |
| 63 | H | i-PrO | H | H | i-PrO | H | H | H |
| 64 | H | n-BuO | H | H | n-BuO | H | H | H |
| 65 | H | i-BuO | H | H | i-BuO | H | H | H |
| 66 | H | s-BuO | H | H | s-BuO | H | H | H |
| 67 | H | t-BuO | H | H | t-BuO | H | H | H |
| 68 | H | OH | H | H | OH | H | H | H |
| 69 | H | -NH₂ | H | H | -NH₂ | H | H | H |
| 70 | H | -NHMe | H | H | -NHMe | H | H | H |
| 71 | H | -NMe₂ | H | H | -NMe₂ | H | H | H |
| 72 | H | -NHEt | H | H | -NHEt | H | H | H |
| 73 | H | -NEt₂ | H | H | -NEt₂ | H | H | H |
| 74 | H | -CH₂OCH₃ | H | H | -CH₂OCH₃ | H | H | H |
| 75 | H | -OCH₂OCH₃ | H | H | -OCH₂OCH₃ | H | H | H |
| 76 | H | -(CH₂)₂O(CH₂)₂CH₃ | H | H | -(CH₂)₂O(CH₂)₂CH₃ | H | H | H |
| 77 | H | -O(CH₂)₂O(CH₂)₂-CH₃ | H | H | -O(CH₂)₂O(CH₂)₂CH₃ | H | H | H |
| 78 | H | Me | H | H | H | H | H | H |
| 79 | H | Et | H | H | H | H | H | H |
| 80 | H | Pr | H | H | H | H | H | H |
| 81 | H | i-Pr | H | H | H | H | H | H |
| 82 | H | n-Bu | H | H | H | H | H | H |
| 83 | H | i-Bu | H | H | H | H | H | H |
| 84 | H | t-Bu | H | H | H | H | H | H |
| 85 | H | MeO | H | H | H | H | H | H |
| 86 | H | EtO | H | H | H | H | H | H |
| 87 | H | PrO | H | H | H | H | H | H |
| 88 | H | i-PrO | H | H | H | H | H | H |
| 89 | H | n-BuO | H | H | H | H | H | H |
| 90 | H | i-BuO | H | H | H | H | H | H |
| 91 | H | s-BuO | H | H | H | H | H | H |
| 92 | H | t-BuO | H | H | H | H | H | H |
| 93 | H | OH | H | H | H | H | H | H |
| 94 | H | -NH₂ | H | H | H | H | H | H |
| 96 | H | -NHMe | H | H | H | H | H | H |
| 96 | H | -NMc₂ | H | H | H | H | H | H |
| 97 | H | -NHEt | H | H | H | H | H | H |
| 98 | H | -NEt₂ | H | H | H | H | H | H |
| 99 | H | -CH₂OCH₃ | H | H | H | H | H | H |
| 100 | H | -OCH₂OCH₃ | H | H | H | H | H | H |
| 101 | H | -(CH₂)₂O(CH₂)₂CH₃ | H | H | H | H | H | H |
| 102 | H | -O(CH₂)₂O(CH₂)₂CH₃ | H | H | H | H | H | H |

### [Table 3]

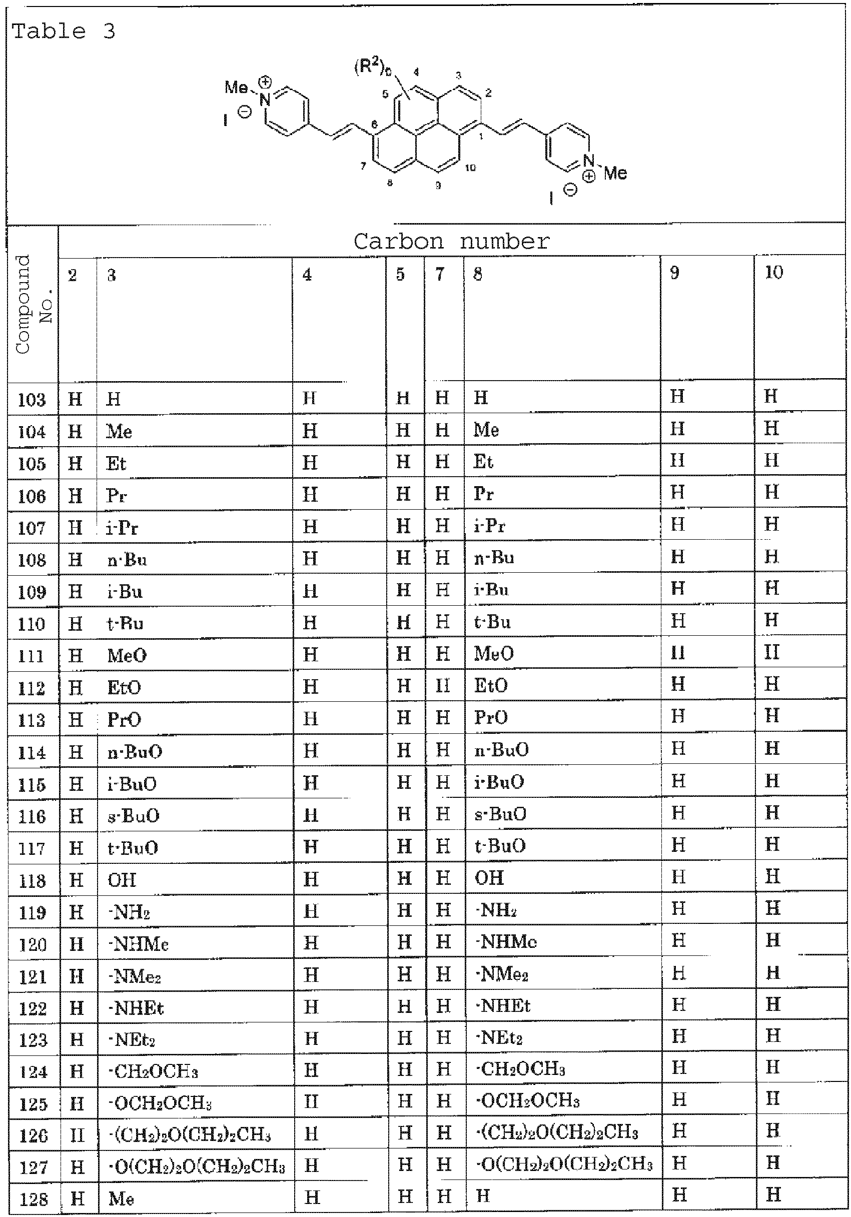

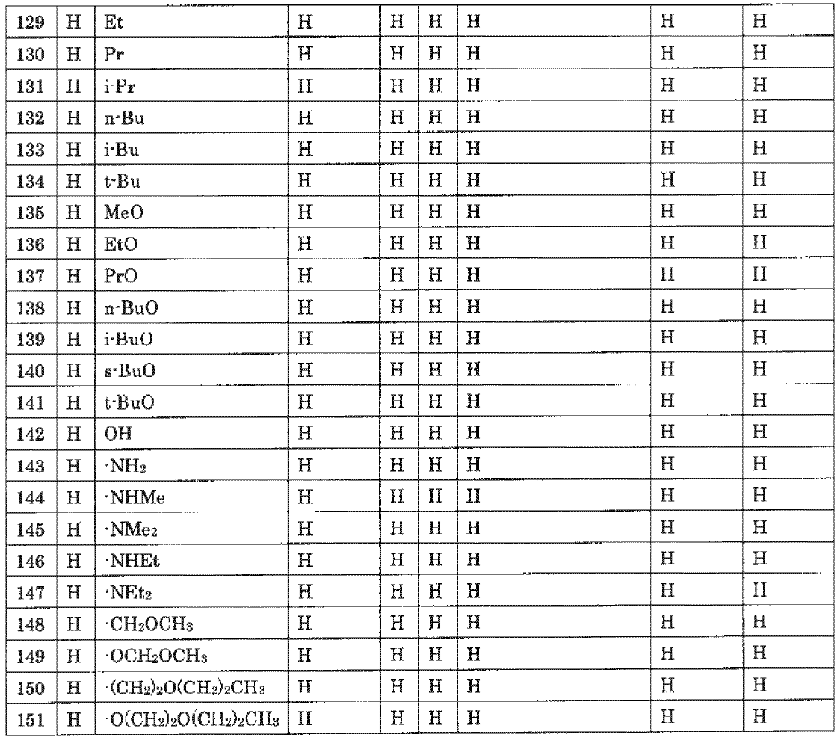

### (Synthesis of compound)

A method for synthesizing the compound represented by formula (1) of the present invention is not particularly limited. Examples of the synthetic method include methods of coupling a condensed polycyclic portion with a pyridine portion via a double bond, as shown in the following Methods 1 to 3.

### Method 1

The coupling of a condensed polycyclic portion with a pyridine portion can be carried out by a Heck reaction. That is to say, an aryl halide represented by formula (3) is reacted with 4-vinylpyridine, as necessary, in a suitable reaction solvent, in the presence of a palladium catalyst and a base, to obtain a compound represented by formula (4). Thereafter, an N-alkylating agent (R¹Z) is added to the compound of formula (4), as necessary, in a suitable reaction solvent, so that the nitrogen in the pyridine is alkylated by the N-alkylating agent to synthesize the compound represented by formula (1). 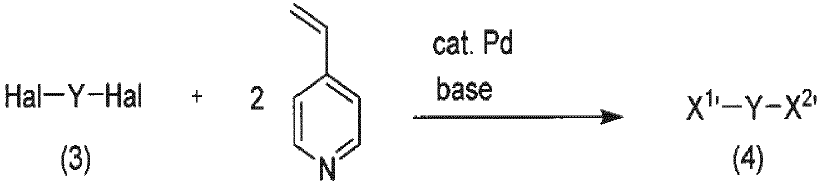 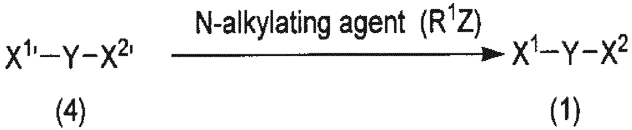 [wherein X¹, X², Y, R¹, and Z are the same as those described above; Hal represents a halogen atom; and X¹' and X²' each represents the following formula: 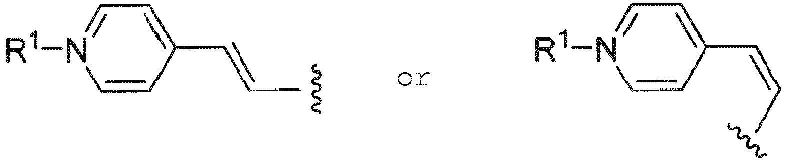 (wherein R¹ and a wavy line are the same as those in formula (1))].

Commercially available products can be used as the above described aryl halide and 4-vinylpyridine. Moreover, the use amount ratio between the above described aryl halide and the above described 4-vinylpyridine compound is not particularly limited. The equivalent ratio of the 4-vinylpyridine to the aryl halide is appropriately selected from the range of 2.0 to 4.0, and preferably of 2.1 to 3.0.

The above described palladium catalyst is not particularly limited, as long as it is a palladium catalyst generally used in a Heck reaction. Examples of the palladium catalyst include palladium acetate, palladium chloride, tris(dibenzylideneacetone)dipalladium, bis(dibenzylideneacetone)palladium, tetrakis(triphenylphosphine)palladium, [1,1'-bis(diphenylphosphino)ferrocene]dichloropalladium, bis(tri-ortho-tolylphosphine)palladium dichloride, bis(triphenylphosphine)palladium dichloride, palladium acetylacetonate, palladium carbon, dichlorobis(acetonitrile)palladium, bis(benzonitrile)palladium chloride, (1,3-diisopropylimidazol-2-ylidene)(3-chloropyridyl)palladium dichloride, bis(tri-tert-butylphosphine)palladium, dichlorobis(triphenylphosphine)palladium(II), and dichlorobis(tricyclohexylphosphine)palladium. The amount of the catalyst used is not particularly limited. The equivalent ratio of the catalyst to the aryl halide is appropriately selected from the range of 0.01 to 0.5, and preferably of 0.05 to 0.3.

The above described base is not particularly limited, as long as it is a base generally used in a Heck reaction. Examples of the base include: amines, such as trimethylamine, triethylamine, diisopropylethylamine, dicyclohexylamine, ethanolamine, diethanolamine, triethanolamine, ethylenediamine, or pyridine; and inorganic bases, such as sodium carbonate, potassium carbonate, cesium carbonate, sodium hydroxide, potassium hydroxide, or cesium hydroxide. The amount of the base used is not particularly limited. The equivalent ratio of the base to the aryl halide is appropriately selected from the range of 2 to 20, and preferably of 3 to 10.

Examples of the solvent used in the coupling reaction between the above described aryl halide and the above described 4-vinylpyridine include: an aromatic hydrocarbon solvent such as benzene or toluene; an amide solvent such as acetonitrile, N,N-dimethylacetamide, or N,N-dimethylformamide; and an ether solvent such as tetrahydrofuran or diethyl ether. These reaction solvents can be used singly, or in an appropriate combination of two or more types of solvents. The amount of the solvent used is not particularly limited. The amount of the solvent used is selected, as appropriate, from an amount range in which the concentration of the aryl halide can be 0.1 to 2 (mol/L), and preferably, 0.5 to 1.5 (mol/L).

The temperature applied during the coupling reaction between the aryl halide and the 4-vinylpyridine is generally 0 to 200°C, and preferably 20 to 130°C. The temperature is selected, as appropriate, depending on the boiling point of a solvent or a base used. The reaction can be carried out in an air atmosphere, but in general, it is preferably carried out in an inert gas atmosphere. Examples of the inert gas include argon, helium, and nitrogen gas.

The reaction solution obtained in the above described coupling reaction is concentrated, as necessary, and then, the residue can be directly used in the subsequent reaction, or the residue can be subjected to an appropriate post-treatment and can be then used as a compound represented by formula (4). Specific examples of the post-treatment method include known purifications such as extraction treatment and/or crystallization, recrystallization, or chromatography.

The above described alkylating agent is not particularly limited, as long as it is an N-alkylating agent generally used in the alkylation of nitrogen. Examples of the alkylating agent include iodomethane, iodoethane, 1-iodopropane, dimethyl sulfate, and methyl trifluoromethanesulfonate. The amount of the alkylating agent used is not particularly limited. The equivalent ratio of the alkylating agent to the compound represented by formula (4) is selected, as appropriate, from the range of 1 to 10, and preferably of 1 to 5.

Examples of a solvent used in the above described alkylation include: an aromatic hydrocarbon solvent such as benzene or toluene; an amide solvent such as acetonitrile, N,N-dimethylacetamide, or N,N-dimethylformamide; an ether solvent such as tetrahydrofuran or diethyl ether; and a halogenated solvent such as dichloromethane, dichloroethane, or chloroform. These reaction solvents can be used singly, or in an appropriate combination of two or more types of solvents. The amount of the solvent used is not particularly limited. The amount of the solvent used is selected, as appropriate, from an amount range in which the concentration of the compound represented by formula (4) can be 0.01 to 2 (mol/L), and preferably, 0.05 to 1.0 (mol/L).

The temperature applied during the above described alkylation reaction is generally 0 to 200°C, and preferably 20 to 130°C. The temperature is selected, as appropriate, depending on the boiling point of a solvent or a base used. The reaction can be carried out in an air atmosphere, but in general, it is preferably carried out in an inert gas atmosphere. Examples of the inert gas include argon, helium, and nitrogen gas.

After completion of the above described alkylation reaction, the reaction solution is concentrated, as necessary, and the precipitated crystal can be directly used, or it can be subjected to an appropriate post-treatment and can be then used as a compound represented by formula (1). Specific examples of the post-treatment method include known purifications such as extraction, crystallization, recrystallization, or chromatography.

### Method 2

Aldehyde represented by formula (5) is reacted with an N-alkyl-4-methylpyridin-1-ium compound represented by formula (6) in the presence of a catalytic amount of base, and as necessary, in a suitable reaction solvent, so as to synthesize the compound represented by formula (1). 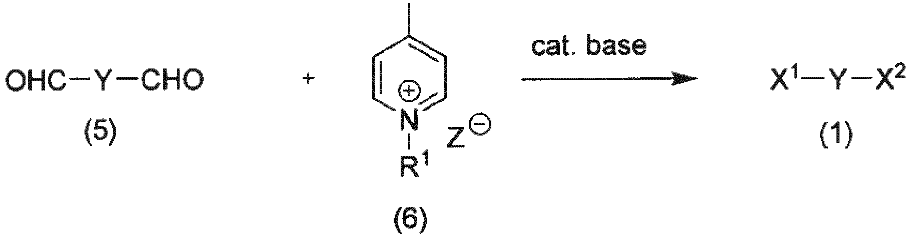 (wherein R¹, X¹, X², Y, and Z⁻ are the same as those described above).

The above described aldehyde can be induced from an aryl compound according to a known method. Examples of the induction method include: a method of inducing aldehyde from an aryl compound, which comprises a reaction of lithiating commercially available aryl halide and then formylating the reaction product; a method of inducing aldehyde from a commercially available aryl compound such as naphthalene, anthracene or pyrene according to a Friedel-Crafts reaction; and a method of inducing aldehyde from a bis(hydroxymethyl)aryl compound by subjecting the compound to a suitable oxidation reaction, but the examples are not limited thereto. The above described N-alkyl-4-methylpyridin-1-ium compound can be synthesized from 4-methyliodopyridine according to the method described in Zhang, Y.; Wang, J.; Ji, P.; Yu, X.; Liu, H.; Liu, X.; Zhao, N.; Huang, B. Org. Biomol. Chem. 2010, 8, 4582-4588, but the synthetic method is not limited thereto. Moreover, the use amount ratio between the above described aldehyde and the above described N-alkyl-4-methylpyridin-1-ium compound is not particularly limited. The equivalent ratio of the N-alkyl-4-methylpyridin-1-ium compound to the aldehyde is appropriately selected from the range of 2.0 to 4.0, and preferably of 2.1 to 3.0.

The above described base is not particularly limited. Examples of the base include trimethylamine, triethylamine, diisopropylethylamine, dicyclohexylamine, ethanolamine, diethanolamine, triethanolamine, ethylenediamine, pyridine, and piperidine. The amount of the base used is not particularly limited. The equivalent ratio of the base to the aldehyde is appropriately selected from the range of 0.01 to 1.0.

Examples of the reaction solvent used in the above described reaction include: an aromatic hydrocarbon solvent such as benzene or toluene; an amide solvent such as acetonitrile, N,N-dimethylacetamide, or N,N-dimethylformamide; an ether solvent such as tetrahydrofuran or diethyl ether; an alcohol solvent such as methanol, ethanol, or isopropanol; and a halogenated solvent such as dichloromethane, dichloroethane, or chloroform. These reaction solvents can be used singly, or in an appropriate combination of two or more types of solvents. The amount of the solvent used is not particularly limited. The amount of the solvent used is selected, as appropriate, from an amount range in which the concentration of the above described aldehyde can be 0.001 to 1.0 (mol/L).

The temperature applied during the above described reaction is generally 0 to 200°C, and preferably 20 to 130°C. The temperature is selected, as appropriate, depending on the boiling point of a solvent or a base used. The reaction can be carried out in an air atmosphere, but in general, it is preferably carried out in an inert gas atmosphere. Examples of the inert gas include argon, helium, and nitrogen gas.

After completion of the reaction, the reaction solution is concentrated, as necessary, and the precipitated crystal can be directly used, or it can be subjected to an appropriate post-treatment and can be then used as a compound represented by formula (1). Specific examples of the post-treatment method include known purifications such as extraction, crystallization, recrystallization, or chromatography.

### Method 3

Furthermore, a compound represented by formula (4) can be induced by a Horner-Wadsworth-Emmons reaction. That is to say, a phosphoric acid ester compound (7) is reacted with 4-pyridinecarboxaldehyde, as necessary, in a suitable reaction solvent, in the presence of a base, to obtain the compound of formula (4). Thereafter, nitrogen in pyridine is alkylated with an N-alkylating agent in the same manner as that in Method 1, so as to synthesize the compound of formula (1). 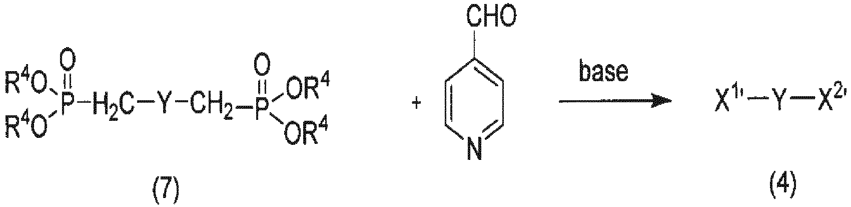 (wherein Y, X¹' and X²' are the same as those described above, and R⁴ represents an ethyl group or a 2,2,2-trifluoroethyl group).

The above described phosphoric acid ester compound represented by formula (7) can be induced by reacting a bis-(halomethyl)aryl compound with triethyl phosphite or tris(2,2,2-trifluoroethyl) phosphite according to the method described in Iwase, Y.; Kamada, K.; Ohta, K.; Kondo, K. J. Mater. Chem. 2003, 13, 1575-1581, but the induction method is not limited thereto. As the above described 4-pyridinecarboxaldehyde, a commercially available product can be used. In addition, the use amount ratio between the above described phosphoric acid ester compound and the above described 4-pyridinecarboxaldehyde is not particularly limited. The equivalent ratio of the 4-pyridinecarboxaldehyde to the phosphoric acid ester compound is appropriately selected from the range of 2.0 to 4.0.

The above described base is not particularly limited. Examples of the base include 1,8-diazabicyclo[5.4.0]-7-undecene, sodium hydride, sodium hexamethyldisilazide, potassium hexamethyldisilazide, benzyltrimethylammonium hydroxide, and tert-butoxypotassium. The amount of the base used is not particularly limited. The equivalent ratio of the base to the aryl halide is appropriately selected from the range of 2.0 to 4.0.

Examples of the reaction solvent used in the above described reaction include: an aromatic hydrocarbon solvent such as benzene or toluene; an amide solvent such as acetonitrile, N,N-dimethylacetamide, or N,N-dimethylformamide; an ether solvent such as tetrahydrofuran or diethyl ether; an alcohol solvent such as methanol, ethanol, isopropanol, or tert-butanol; and a halogenated solvent such as dichloromethane, dichloroethane, or chloroform. These reaction solvents can be used singly, or in an appropriate combination of two or more types of solvents. The amount of the solvent used is not particularly limited. The amount of the solvent used is selected, as appropriate, from an amount range in which the concentration of aldehyde can be 0.001 to 1.0 (mol/L).

The temperature applied during the above described reaction is generally 0 to 200°C, and preferably 20 to 130°C. The temperature is selected, as appropriate, depending on the boiling point of a solvent or a base used. The reaction can be carried out in an air atmosphere, but in general, it is preferably carried out in an inert gas atmosphere. Examples of the inert gas include argon, helium, and nitrogen gas.

After completion of the reaction, the reaction solution is concentrated, as necessary, and the precipitated crystal can be directly used, or it can be subjected to washing or an appropriate post-treatment and can be then used as a compound represented by formula (1). Specific examples of the post-treatment method include known purifications such as extraction, crystallization, recrystallization, or chromatography.

### (Fluorescent probe composition)

The above described compound represented by formula (1) can be directly used as a fluorescent probe. However, as necessary, additives generally used in preparation of reagents can be mixed with the compound of formula (1), and the obtained mixture can be used as a fluorescent probe composition. For example, as additives for the use of a reagent in a physiological environment, additives such as a solubilizer, a pH adjuster, a buffer and a tonicity agent can be used. The amount of these additives mixed can be appropriately determined by a person skilled in the art. Such a composition is generally provided in an appropriate form such as a powdery mixture, a freeze-dried product, a granule, a tablet, or a liquid agent.

### (Bioimaging)

The bioimaging of the present invention is carried out by the following steps:
1) a step of administering the compound represented by formula (1) or a fluorescent probe composition comprising the above described compound to cells, tissues, an organ or an individual body;
2) a step of distributing the above described compound into the above described cells, tissues, organ or individual body, and then allowing the compound to come into contact with biomolecules in the above described cells, tissues, organ or individual body;
3) a step of exposing the above described cells, tissues, organ or individual body to a light with a wavelength that can be absorbed by the above described compound; and
4) a step of detecting fluorescence released from the above described compound.

In the step 1), when the above described compound or fluorescent probe composition is administered to cells, tissues, an organ or an individual body, it can be dissolved in a solvent such as water or dimethyl sulfoxide, or in a buffer. For instance, when the administration targets are cells, there is applied a method which comprises mixing the above described compound or fluorescent probe composition into a medium in which the cells are cultured, for example, but the applied method is not limited thereto.

In the step 2), examples of the above described biomolecule include a nucleic acid, a protein, and a phospholipid, which are present in a nucleus, an endoplasmic reticulum, a Golgi body, an endosome, a lysosome, mitochondria, a chloroplast, a peroxisome, a cell membrane, and a cell wall. In addition, by allowing the above described compound to come into contact with such a biomolecule, a chemical bond, such as a covalent bond, an ionic bond, a coordination bond, a hydrogen bond or a van der Waals bond, is formed, so that a biomolecule exhibiting a fluorescent property can be obtained. In addition, in the step 2), it can be preferably demonstrated the above described compound and a biomolecule existing in mitochondria form a chemical bond.

In the step 3), the wavelength that can be absorbed by the above described compound is not particularly limited, as long as it is an ultraviolet region, a visible region, or an infrared region. In order to obtain an image of the deep part of a cell, a tissue, an organ or an individual body, a wavelength region at 600 to 1200 nm is preferable because it has high permeability into them. As a light source for excitation light, a commercially available light source can be used. Moreover, as a method of exposing the cell, tissue, organ or individual body to light, it is preferable to concentrate the excitation light in the wavelength region at 600 to 1200 nm with lens or the like and to scan the position of a focal point, so as to obtain a three-dimensional image of the cell, tissue, organ or individual body by utilizing two-photon absorption of the above described compound.

The step 3) and the step 4) can be carried out by two-photon excitation fluorescence microscopy, as described in Example 5 of the present invention.

### Examples

Hereinafter, the present invention will be described more in detail in the following examples. However, these examples are not intended to limit the technical scope of the present invention.

### Example 1. Synthesis of naphthalene derivative (I)

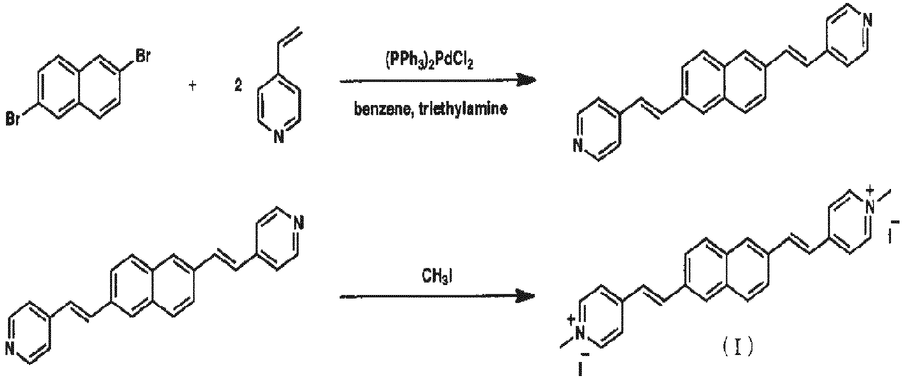

To a Schlenk tube in an argon atmosphere, 2,6-dibromonaphthalene (0.29 g, 1 mmol) and dichlorobis(triphenylphosphine) palladium(II) (Pd(PPh₃)₂Cl₂, 0.077 g, 0.11 mmol) were added. To the tube, 4-vinylpyridine (0.26 g, 2.56 mmol), 1 mL of benzene, and 1 mL of triethylamine were added, and the thus obtained mixture was then stirred at 100°C under heating for 3 days. Thereafter, the mixed solution was filtrated and concentrated. Thereafter, the concentrate was extracted with dichloromethane, and the organic layer was then washed with water. Magnesium sulfate (anhydrous) was added to the organic layer, and then, the obtained mixture was dried and concentrated. Crude crystals were recrystallized from acetone to obtain 4,4-(2,6-naphthalenediyldi-(1E)-2,1-ethenediyl)bispyridine in the form of a golden solid.

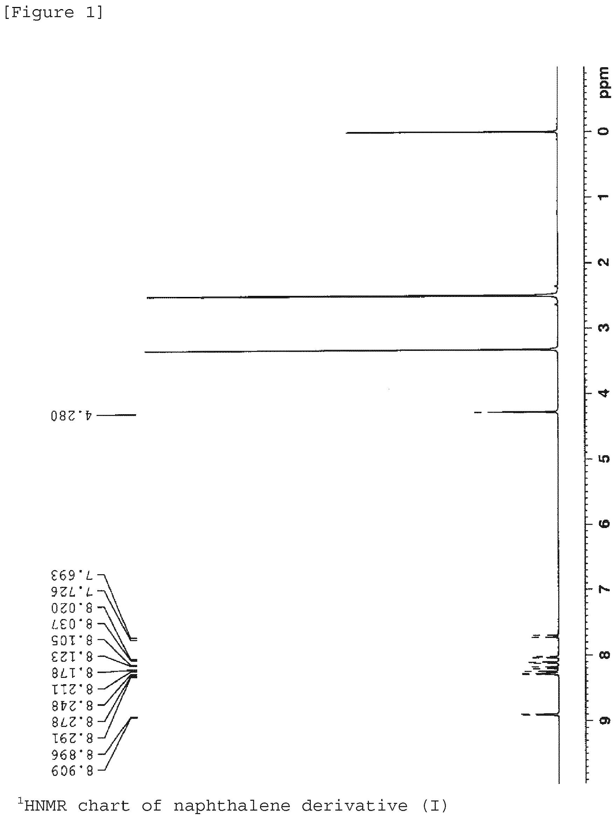

The 4,4-(2,6-naphthalenediyldi-(1E)-2,1-ethenediyl)bispyridine (0.33 g, 1 mmol) was dissolved in 10 mL of dichloromethane, and iodoethane (CH₃I, 1 mL) was then added to the solution, followed by stirring the mixture at room temperature for 24 hours. Thereafter, the precipitated solid was washed with dichloromethane to obtain a naphthalene derivative (I) in the form of a yellow solid. The ¹HNMR of the obtained naphthalene derivative (I) is shown in Figure 1.

### Example 2. Synthesis of anthracene derivative (II)

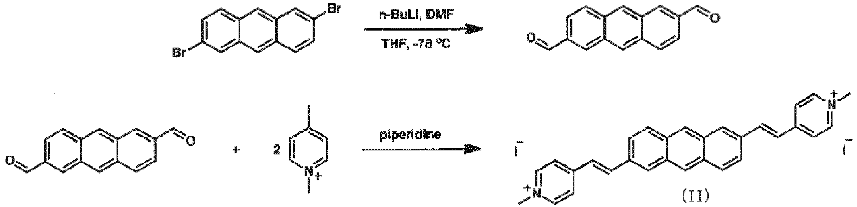

To a flame-dried two-necked flask, 2,6-dibromoanthracene (0.34 g, 1 mmol) was added, and thereafter, the inside of the container was filled with argon gas. Anhydrous tetrahydrofuran (15 mL) was added to the flask, and the mixed solution was then cooled to -78°C. To the reaction solution, n-butyllithium (n-BuLi, 21.4 mL, 34.2 mmol) was slowly added dropwise, and the obtained mixture was then stirred at -78°C for 1 hour. Thereafter, anhydrous dimethylformamide (DMF) was slowly added dropwise to the reaction solution, and the obtained mixture was then stirred for 1 hour. Thereafter, the temperature was slowly increased to room temperature, and water was then added to the reaction solution for quenching. The resultant was extracted with toluene, and the organic layer was then washed with water. After that, magnesium sulfate (anhydrous) was added to the organic layer, so that the obtained mixture was dried and concentrated. The concentrate was purified by column chromatography (developing solvent = chloroform 9 : acetone 1). Thereafter, the resultant was recrystallized from a mixed solvent of toluene/ethanol, to obtain anthracene-2,6-dicarbaldehyde in the form of a yellow solid.

1,4-Dimethylpyridin-1-ium iodide was synthesized by the previously reported method (Zhang, Y.; Wang, J.; Ji, P.; Yu, X.; Liu, H.; Liu, X.; Zhao, N.; Huang, B. Org. Biomol. Chem. 2010, 8, 4582-4588.).

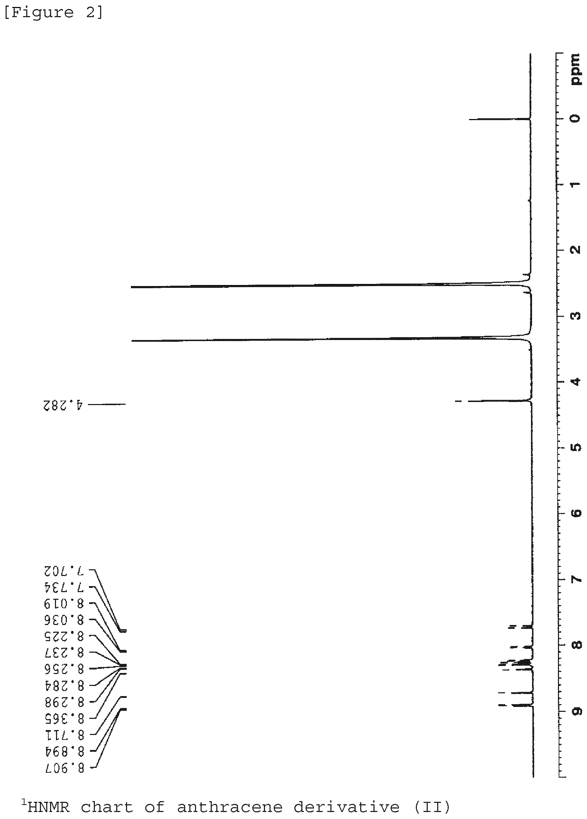

To the flask, anthracene-2,6-dicarbaldehyde (0.04 g, 0.17 mmol) and 1,4-dimethylpyridinium iodide (0.07 g, 0.3 mmol) were added, and the obtained mixture was then dissolved in 20 mL of ethanol. 10 Droplets of piperidine were added dropwise to the solution, and the thus obtained mixture was stirred at 80°C under heating for 24 hours. The precipitated solid was filtrated and was then washed with ethanol to obtain an anthracene derivative (II) in the form of an orange solid. The ¹HNMR of the obtained anthracene derivative (II) is shown in Figure 2.

### Example 3. Synthesis of pyrene derivative (III)

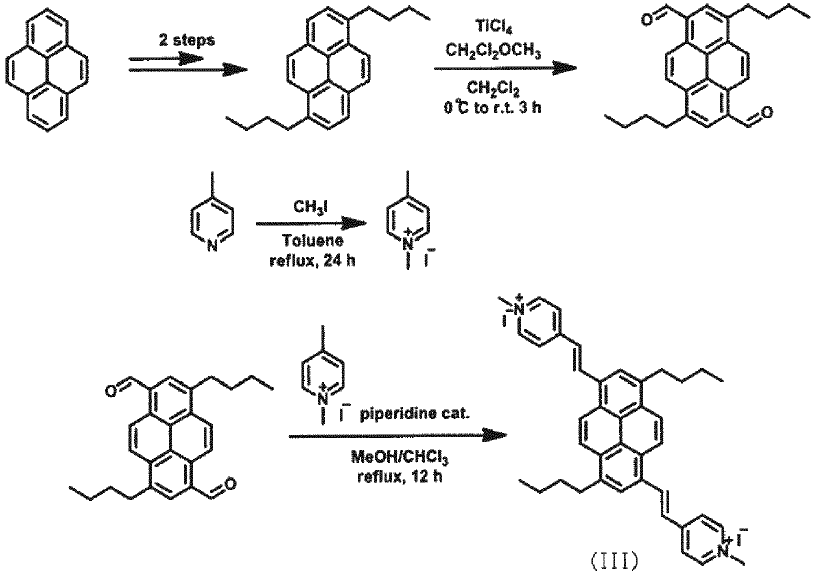

1,6-Dibutylpyrene was synthesized, using pyrene as a starting substance, according to the previously reported method (Minabe, M.; Takeshige, S.; Soeda, Y.; Kimura, T.; Tsubota, M. Bull. Chem. Soc. Jpn. 1994, 67, 172-179. and Niko, Y.; Kawauchi, S.; Otsu, S.; Tokumaru, K.; Konishi, G. J. Org. Chem. 2013, 78, 3196-3207.).

To 5 mL of dichloromethane, 1,6-dibutylpyrene (0.33 g, 1.06 mmol) and dichloromethyl methyl ether (0.50 mL, 5.3 mmol) were dissolved, and the obtained solution was then cooled to 0°C. To the reaction solution, a solution prepared by dissolving titanium tetrachloride (0.6 mL, 5.3 mmol) in 2 mL of dichloromethane was added. The reaction solution was stirred at room temperature for 24 hours. Thereafter, the reaction was quenched by a large amount of ice water, and it was then extracted with chloroform. The obtained organic layer was washed with a sodium hydrogen carbonate aqueous solution and a saline, and was then dried over anhydrous magnesium sulfate (MgSO₄) and concentrated. The crude product was purified by column chromatography (chloroform : hexane = 3:1) and the subsequent recrystallization from methanol, so as to obtain 3,8-dibutylpyrene-1,6-dicarbaldehyde (0.16 g, yield: 41%) in the form of a yellow solid.

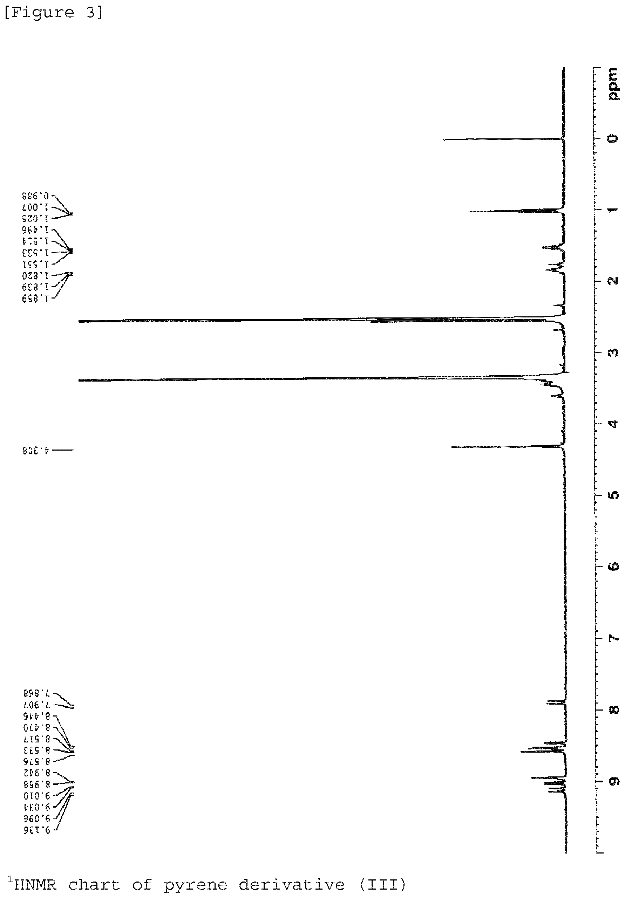

3,8-Dibutylpyrene-1,6-dicarbaldehyde (0.10 g, 0.27 mmol) and 1,4-dimethylpyridin-1-ium iodide (0.16 g, 0.67 mmol) were dissolved in a mixed solution consisting of 10 mL of chloroform and 30 mL of methanol, and a catalytic amount of piperidine (5 droplets) was then added dropwise to the obtained solution. The reaction solution was refluxed for 12 hours. The reaction solution was concentrated, and the obtained crude product was then washed with hot methanol and hot chloroform twice, so as to obtain a pyrene derivative (0.13 g, yield: 59%) in the form of a red solid. The ¹HNMR of the obtained pyrene derivative (III) is shown in Figure 3.

### Comparative Example 1. Benzene derivative (IV)

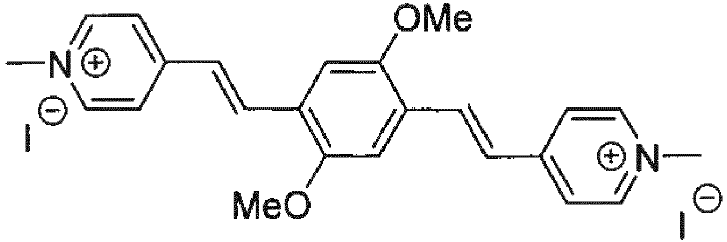

A benzene derivative (IV) was synthesized according to the method described in Iwase, Y.; Kamada, K.; Ohta, K.; Kondo, K. J. Mater. Chem. 2003, 13, 1575-1581.

### Example 4. Measurement of ultraviolet-visible absorption spectrum, fluorescence spectrum, and two-photon absorption cross-section

The ultraviolet-visible absorption spectrum, fluorescence spectrum, and two-photon absorption cross-section of the compounds synthesized in Examples 1-3 and Comparative Example 1 were measured under the following conditions.

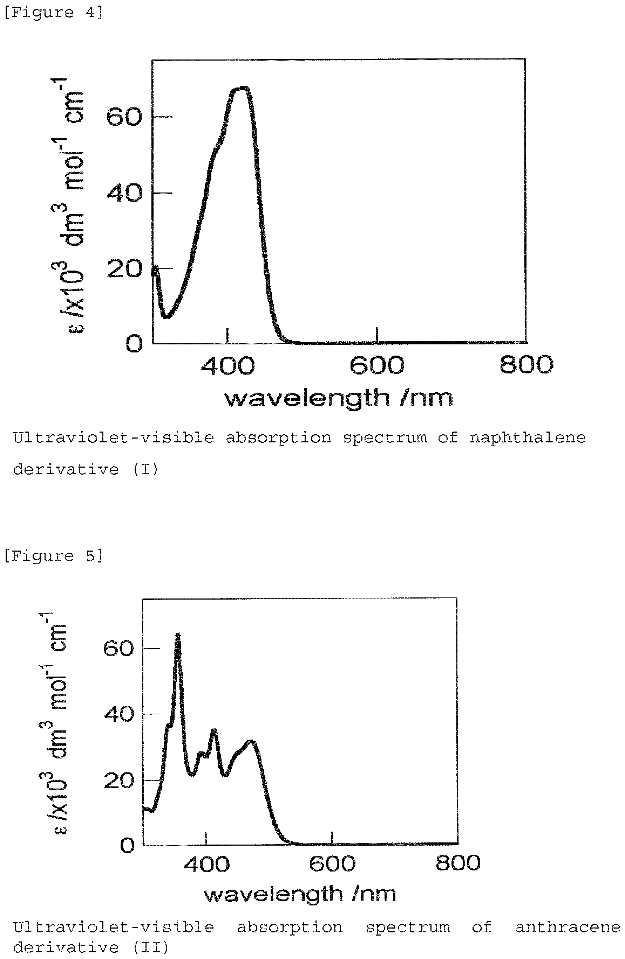

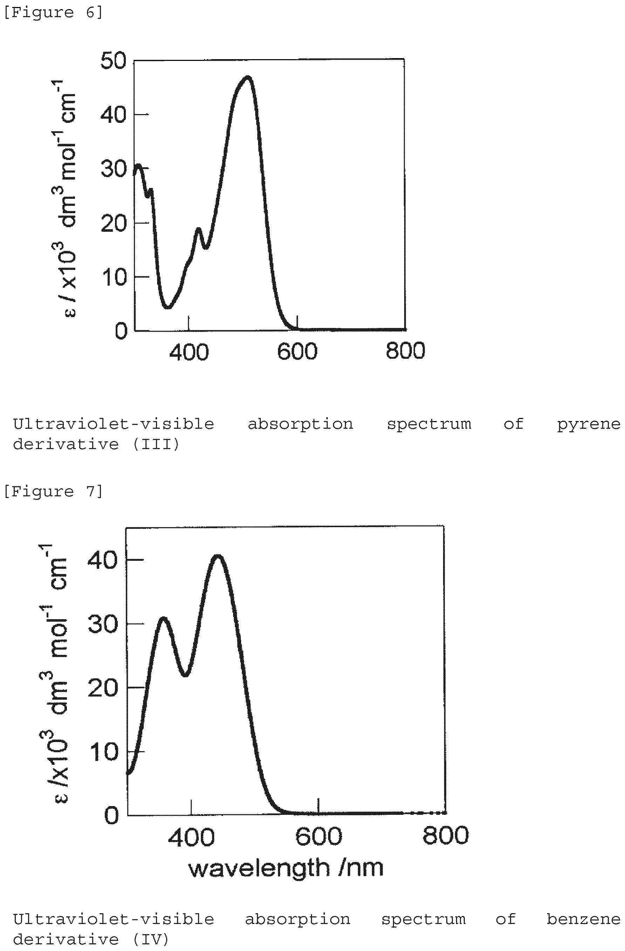

The ultraviolet-visible absorption spectrum was measured using V-670-UV-VIS-NIR spectrophotometer (Jasco Co.). The measurement results are shown in Figure 4 to Figure 7.

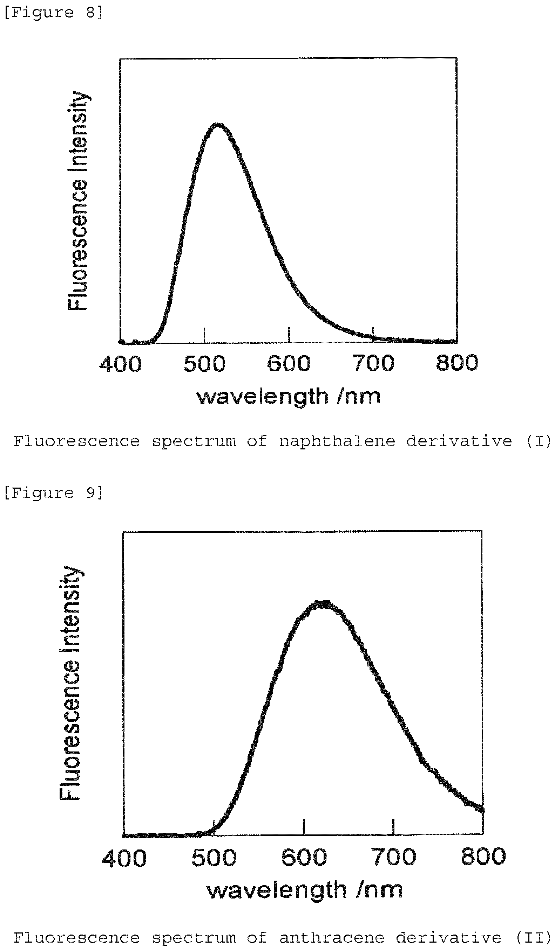

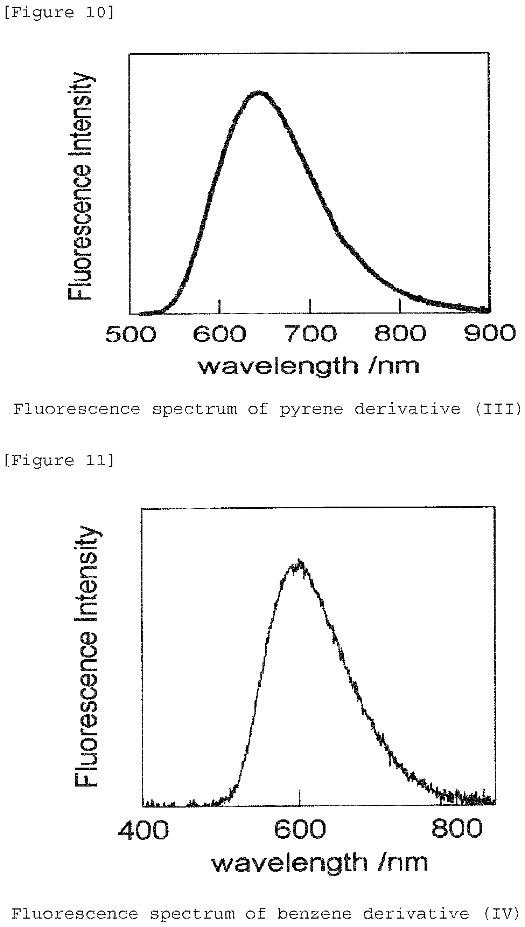

The fluorescence spectrum was measured using C9920-03G (Hamamatsu Photonics. K. K.). The fluorescence quantum yield was determined by absolute measurement using an integrating sphere. The measurement was carried out using a sample that had been adjusted to have a concentration of 10⁻⁶ mol/L. The measurement results are shown in Figure 8 to Figure 11.

The two-photon absorption cross-section was determined according to the following procedures. Since two-photon absorption behavior has a spectroscopic property, as with one-photon absorption behavior, in order to compare substances in terms of two-photon absorption cross-section, it is necessary to measure spectra. Thus, two-photon absorption cross-sections were measured at several wavelengths, and the obtained values were then plotted against the wavelength on the horizontal axis to prepare a two-photon absorption spectrum. The value of two-photon absorption cross-section at each wavelength was estimated by an open aperture Z scan method. As a light source, a laser light, which was obtained by wavelength conversion of a laser light outputted from a regenerative amplifier (Spectra-Physics, Spitfire) using a difference frequency generation device (Spectra-Physics, OPA-800C), was used.

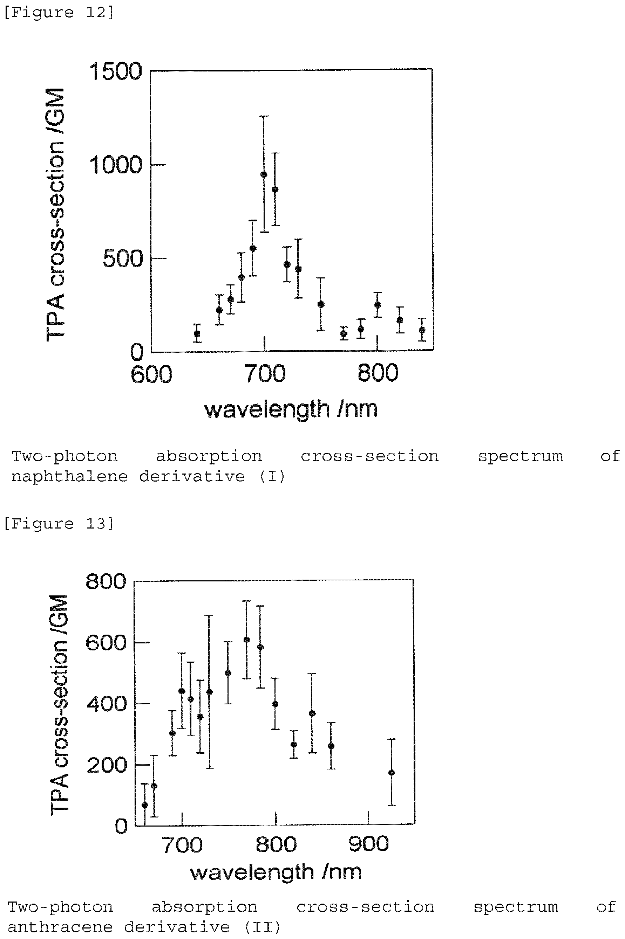

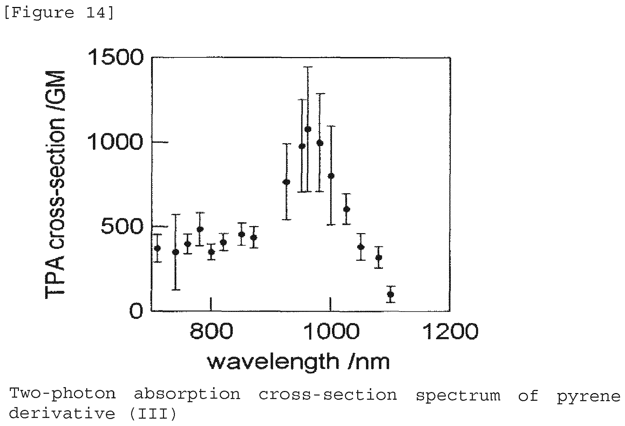

The repetition frequency of the outputted laser light was 1 kHz, and the pulse width was 150 to 200 fs. The two-photon absorption cross-section was estimated based on the degree of a change in permeability, which was observed when a laser light was concentrated with lens having a focal distance of 15 cm and then moving a sample along the optical axis. The average power of the used laser lights was 0.01 to 0.4 mW, and the peak output was 6 to 240 GW/cm². The spectra of two-photon absorption cross-sections are shown in Figures 12 to 15.

### Example 5. Fluorescence staining experiment on cells and observation thereof

### Culture of cells

Human embryonic kidney cells, namely, Hek293 cells were used as model cells for staining. The Hek293 cells were cultured in Dulbecco's Modified Eagle's Medium (DMEM) containing 10% (v/v) fetal bovine serum and 1% (v/v) trypsin and streptomycin under conditions of 37°C and 5%CO₂.

### Fluorescence staining of cells

For preparation of microscopic observation, Hek293 cells were subcultured on a 35-mm glass-based dish, to result in a cell density of 1 × 10⁵ cells/dish. 24 hours after the subculture, adhesion of the cells to the dish was confirmed by microscopic observation. The medium was removed from the dish, and the resulting cells were then washed with a phosphate buffered saline (PBS) twice. 2 mL of DMEM medium not containing phenol red (final concentration of PY: 1 µmol dm⁻³, final DMSO concentration: 0.1% (v/v)), to which 2 µL of dimethyl sulfoxide (DMSO) solution of the 1 × 10⁻³ mol/dm⁻³ naphthalene derivative (I), anthracene derivative (II) or pyrene derivative (III) had been added, was placed in the dish, and it was then incubated for 12 hours for staining. Immediately before microscopic observation, the medium containing the pigment was removed from the dish, and PBS was then used to wash the cells twice. After that, 2 mL of the DMEM medium not containing phenol red was added to the dish.

### Two-photon excitation fluorescence microscopic observation

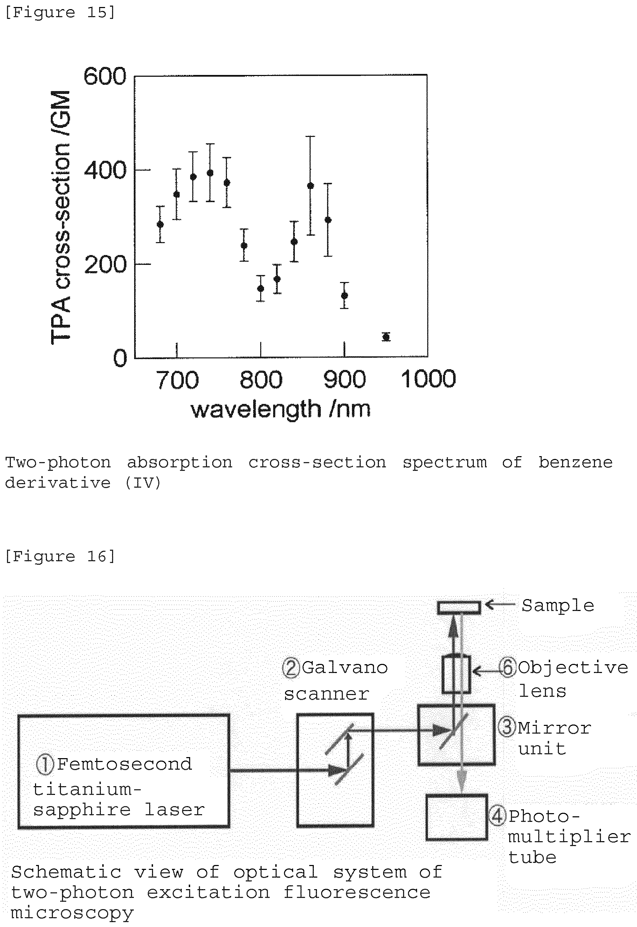

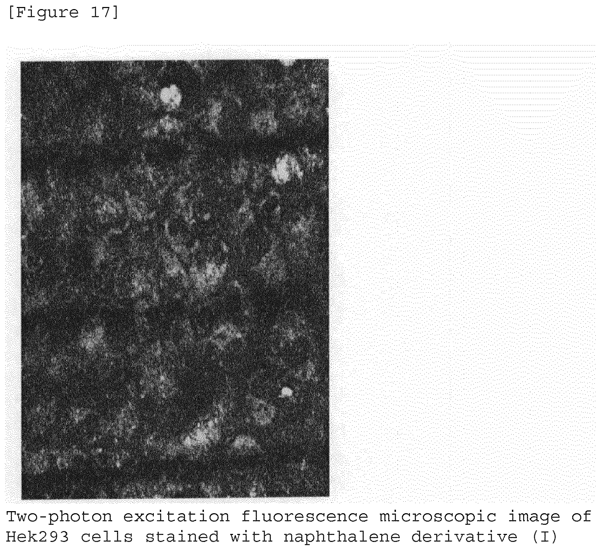

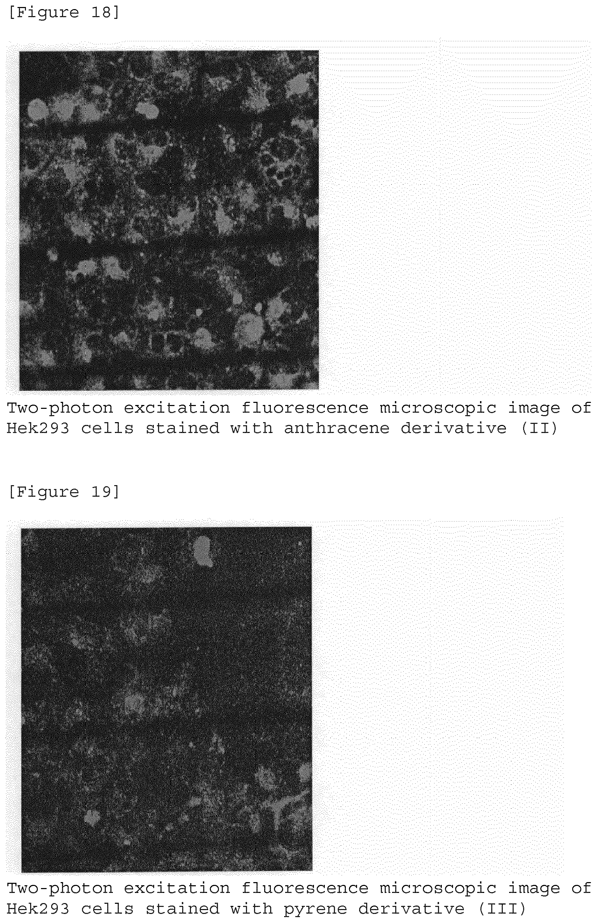

A two-photon excitation fluorescence microscope was produced using Optical Block (Hamamatsu Photonics K. K.). The optical system thereof is shown in Figure 16. As a light source, Femtosecond titanium-sapphire laser (Mira900, Coherent) was used. A mirror unit equipped with a Galvano scanner for scanning a focal point, a short-pass dichroic mirror (FF750-SDi02-25 × 36, Semrock) having a cutoff wavelength at 750 nm, and a band pass filter (FF01-650/60-25, Semrock) having a central wavelength at 650 nm was inserted into the optical system. For detection of fluorescence, a photomultiplier tube (R928, Hamamatsu Photonics K. K.) was used, and DC was detected at an applied voltage of 1000 V, through a preamplifier (5 MHz)-equipped socket. USB-6251 BNC was used as DAQ, and Lab VIEW2011 (National Instruments) was used as a platform of the control program. As a sample stage, KZG0620-G was used, and as objective lens, infinity corrected objective lens with a magnification of 40 and NA of 1.15 was used. The images obtained as a result of observation are shown in Figures 17 to 19.

The naphthalene derivative (I), anthracene derivative (II) and pyrene derivative (III) of the present invention were each dissolved in dimethyl sulfoxide (DMSO), and they could achieve the fluorescence staining of HeK293 cells. In addition, the emission of a red fluorescence from the aforementioned cells was observed by two-photon excitation fluorescence microscopy.

### Industrial Applicability

Since the compound of the present invention is excited by two-photon absorption in a near-infrared wavelength region, emits a red fluorescence, and also has water-solubility, it can be used as a fluorescent probe. The present compound is administered to cells, tissues, an organ and an individual body, so as to obtain their bioimagings. Moreover, since the present compound emits a red fluorescence that easily passes through an organism, it becomes possible to achieve the imaging of the deep part of an organism.

## Claims

1. A compound represented by the following formula (1):
X¹-Y-X² (1)
[wherein X¹ and X² are the same or different, and each represents the following formula (2): 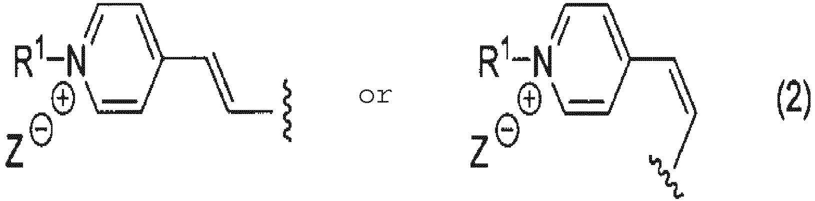 (wherein R¹ represents a C1-C3 alkyl group, Z⁻ represents a counter anion to a pyridinium cation, and a wavy line represents a covalent bond to Y), and Y represents a condensed polycyclic group having 2 to 4 rings].

2. The compound according to claim 1, wherein Y is any one of condensed polycyclic groups represented by the following formulae: 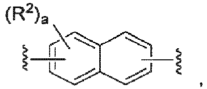 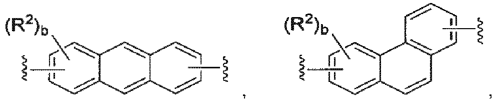 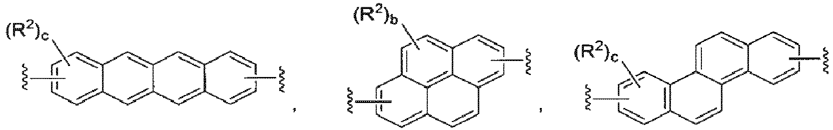 (wherein R² represents an electron-donating group, a represents an integer of 0 to 6, b represents an integer of 0 to 8, and c represents an integer of 0 to 10; when a, b, or c is an integer of 2 or more, R² is identical to or different from one another; and a wavy line represents a covalent bond to X¹ and X²).

3. The compound according to claim 2, wherein Y is any one of condensed polycyclic groups represented by the following formulae: 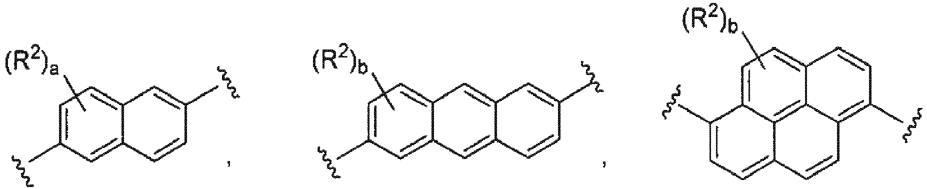 (wherein R² represents an electron-donating group, a represents an integer of 0 to 6, and b represents an integer of 0 to 8; when a or b is an integer of 2 or more, R² is identical to or different from one another; and a wavy line represents a covalent bond to X¹ and X²).

4. The compound according to claim 2 or 3, wherein the electron-donating group is one or more selected from the group consisting of a hydroxyl group, a C1-C10 alkyl group, a C1-C10 alkoxy group, an amino group, an alkyl group having an ether bond, and an alkoxy group having an ether bond.

5. The compound according to any one of claims 1 to 4, wherein the counter anion is a halide ion or sulfonate.

6. The compound according to any one of claims 1 to 5, wherein the compound has two-photon absorption in a wavelength region of 600 to 1200 nm.

7. The compound according to any one of claims 1 to 6, wherein the compound emits fluorescence in a wavelength region of 600 to 900 nm.

8. A fluorescent probe composition comprising one or more of the compounds according to any one of claims 1 to 7.

9. A fluorescent probe composition for use in bioimaging, comprising one or more of the compounds according to any one of claims 1 to 7.

10. A fluorescent biomolecule comprising a biomolecule to which the compound according to any one of claims 1 to 7 chemically binds.
